# EUROPEAN PATENT APPLICATION

(11) **EP 1 767 141 A1**
(43) Date of publication of application: **28.03.2007**
(21) Application number: 05741518.4
(22) Date of filing: 18.05.2005
(51) Int. Cl.: A61B 3/10

(54) **PUPIL DETECTOR AND IRIS IDENTIFICATION DEVICE**

(30) Priority: 14.07.2004 JP 2004206932
(71) Applicant: MATSUSHITA ELECTRIC INDUSTRIAL CO., LTD., Osaka 571-8501 (JP)
(72) Inventor: SUGITA, Morio Matsushita Electric Industrial CO, Osaka 571-8501 (JP); WAKAMORI, Masahiro Matsushita Electric Industrial, Osaka 571-8501 (JP); FUJIMATSU, Takeshi Matsushita Electrical Industri, Osaka 571-8501 (JP)
(74) Representative: Grünecker, Kinkeldey, Stockmair & Schwanhäusser Anwaltssozietät
(86) International application number: PCT/JP2005/009045
(87) International publication number: WO 2006/006298

(57) **Abstract**

A pupil candidate detection unit (210) for detecting positions of pupil candidates which may be candidates of a pupil in an eye image; a pupil candidate retention unit (280) for retaining a plurality of detected positions of the pupil candidates; and a pupil selection unit (290) for selecting a pupil candidate, out of the plurality of pupil candidates, which includes center positions of other pupil candidates in an area within a predetermined distance from the center position of its own are provided.

## Description

### Technical Field

The present invention relates to an iris authentication apparatus used for personal authentication or the like and, more specifically, to a pupil detection device for detecting the position of a pupil from an image including an eye (hereinafter, abbreviated as "eye image"}.

### Background Art

Hitherto, various methods for detecting the position of a pupil from an eye image have been proposed, and for example, a method of binarizing image data of the eye image (hereinafter, abbreviated as "eye image data") and detecting a circular area in an area of low-luminance level, and a method of calculating a contour integral of an image luminance I (x, y) with respect to an arc of a circle having a radius r and center coordinates (x0 , y0 and calculating a partial derivative of the calculated amount relating to r in association with increase in the radius r (for example, JP-T-8-504979) is known. Several methods of increasing detection accuracy by eliminating the effects of eyelash or sunlight have been also disclosed (for example, JP-A-2002-119477).

In order to detect the pupil with high degree of accuracy using these methods, it is necessary to process a huge amount of image data at high-speed, and hence it is difficult to process the image data of the eye image on real time basis even though a large CPU having a high processing capability or a bulk memory in the status quo. Also, when the processing amount of the CPU is reduced to a degree which enables real time processing of the image data, there may arise a problem such that the detection accuracy is lowered.

### Disclosure of the Invention

The invention provides a pupil detection device and an iris authentication apparatus which can detect the position of a pupil at high-speed and with high degree of accuracy.

The pupil detection device of the invention includes a pupil candidate detection unit for detecting positions of pupil candidates which may be candidates of a pupil in an eye image, a pupil candidate retention unit for retaining a plurality of positions of the pupil candidates detected by the pupil candidate detection unit, and a pupil selection unit for selecting a pupil candidate, out of the pupil candidates retained in the pupil candidate retention unit, which includes center positions of other pupil candidates in an area within a predetermined distance from the center position of its own as a pupil.

### Brief Description of the Drawings

Fig. 1 is a circuit block diagram of an iris authentication apparatus using a pupil detection device according to a first embodiment of the invention.
Fig. 2A is a drawing showing an example of an image including a pupil.
Fig. 2B is a drawing showing an integrated value with respect to a radius of an integrating circle.
Fig. 2C is a drawing showing a value obtained by differentiating the integrated value by the radius of the integrating circle.
Fig. 2D is a drawing showing the integrating circles moving on an eye image.
Fig. 3A is a drawing showing an example of the eye image when the integrating circle is positioned in an iris area and luminance at the same moment.
Fig. 3B is a drawing showing an example of the eye image when the integrating circle is positioned on an eyeglass frame and luminance of the same moment.
Fig. 4 is a circuit block diagram of the pupil detection device in the same embodiment.
Fig. 5 is a circuit drawing of an image data extraction unit in the same embodiment.
Fig. 6 is a circuit block diagram of a pupil candidate retention unit and a pupil selection unit in the same embodiment.
Fig. 7 is a drawing for explaining an operation of the pupil selection unit in the same embodiment.
Fig. 8 is a flowchart showing an operation corresponding to one frame of the eye image of the pupil detection device according to the same embodiment.
Fig. 9 is a flowchart for selecting a pupil in pupil candidates in another embodiment of the invention.

### Reference Numerals

- 120: image pickup unit
- 130: illumination unit
- 140: authentication processing unit
- 200: pupil detection device
- 220: image data extraction unit
- 230: contour integrating unit
- 240: luminance difference calculation unit
- 250: pupil radius detection unit
- 260: pointer unit
- 280: pupil candidate retention unit
- 290: pupil selection unit

### Best Mode for Carrying Out the Invention

A pupil detection device of the invention includes a pupil candidate detection unit for detecting positions of pupil candidates which can be candidated of a pupil from an eye image, a pupil candidate retention unit for retaining a plurality of positions of the pupil candidates detected by the pupil candidate detection unit, and a pupil selection unit for selecting a pupil candidate, out of the pupil candidates retained in the pupil candidate retention unit, which includes center positions of other pupil candidates in an area within a predetermined distance from the center position of its own as a pupil. In this arrangement, the pupil detection device which can detect the pupil position at high-speed and with high degree of accuracy is provided.

The pupil detection device of the invention also includes an image data extraction unit for determining a plurality of concentric circles on an eye image as integrating circles respectively, and extracts image data of the eye image positioned on the circumferences of the integrating circles, a contour integrating unit for integrating the image data extracted by the image data extraction unit along the respective circumferences of the integrating circles, a pupil radius detection unit for detecting the integrating circle whose integrated value obtained from the contour integrating unit has changed stepwise with respect to the radius of the integrating circle out of the plurality of integrating circles, the pupil candidate retention unit for retaining the center coordinates of the integrating circle detected by the pupil radius detection unit as the coordinate position of the pupil candidate, and the pupil selection unit for selecting a pupil candidate, out of the pupil candidates retained in the pupil candidate retention unit, which includes center positions of other pupil candidates in a area within an predetermined distance from the center position of its own. In this arrangement, the pupil can be detected in a real time basis with respect to the image data picked up by an image pickup unit.

The pupil radius detection unit of the pupil detection device of the invention outputs a difference of the integrated value of the contour integrating unit stepwise with respect to the radius of the integrating circle as an evaluated value, and the pupil candidate retention unit includes a plurality of maximum value detectors for detecting data having a maximum value out of input data connected in series and inputs the evaluated value in the pupil candidate retention unit, thereby retaining the pupil candidates in the descending order of the evaluated value. In this arrangement, the pupil candidates can be arranged in the descending order using the evaluated value.

The maximum value detector in the pupil detection device of the invention includes a register for retaining input data, a comparator for comparing data retained in the register and input data, and a selector for selecting and outputting one of the data retained in the register or the input data, and may be configured in such a manner that the register retains the input data when the input data is larger than the retained data based on the output of the comparator, and the selector selects the data retained by the register when the input data is larger than the data retained by the register and selects the input data when the input data is smaller than the data retained by the register based on the output of the comparator. In this arrangement, the maximum value detector can be achieved with a relatively simple circuit.

The pupil selection unit of the pupil detection device of the invention may be adapted to sort the plurality of pupil candidates into groups by grouping those close to each other as one group, select a group in which the largest number of pupil candidates are included or a group in which the sum of evaluated values of the pupil candidates are the largest out of the respective groups, and determine the pupil position in the selected group. In this arrangement, the pupil selection unit can be realized using a relatively simple algorithm.

An iris authentication apparatus of the invention is provided with the pupil detection device of the invention. In this arrangement, the iris authentication apparatus in which the pupil detection device which can detect the position of the pupil at high-speed and with high degree of accuracy can be provided.

Referring to the drawings, the iris authentication apparatus in which the pupil detection device in the embodiment of the invention will be described below.

### (First Embodiment)

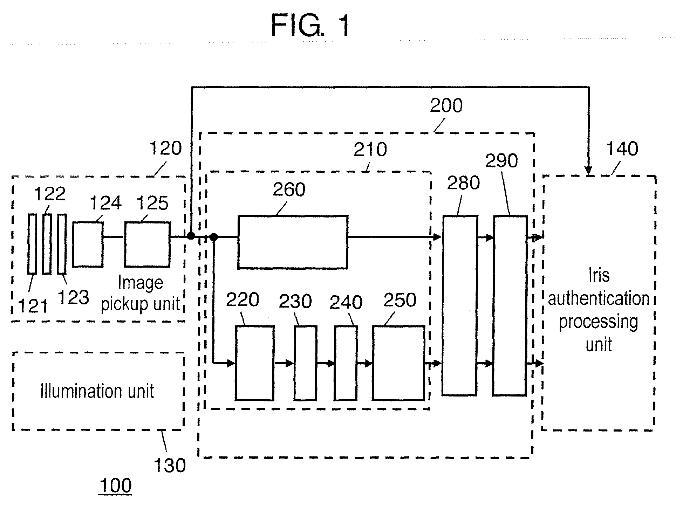

Fig. 1 is a circuit block diagram of iris authentication apparatus 100 in which pupil detection device 200 according to a first embodiment of the invention is employed. In addition to pupil detection device 200, Fig. 1 also illustrates image pickup unit 120, illumination unit 130, authentication processing unit 140 which are necessary to configure iris authentication apparatus 100.

Iris authentication apparatus 100 according to the first embodiment includes image pickup unit 120 for picking up an eye image of a user, pupil detection device 200 for detecting the position of the pupil and the radius thereof from the eye image, authentication processing unit 140 for performing personal authentication by comparing an iris code obtained from the eye image with a registered iris code, and illumination unit 130 for irradiating near-infrared ray of a light amount suitable for obtaining the eye image for illuminating the user' s eye and the periphery thereof.

Image pickup unit 120 includes guide mirror 121, visible light eliminating filter 122, lens 123, image pickup element 124 and preprocessing unit 125. In this embodiment, by using a fixed focal length lens as lens 123, compact and light weighted optical system and cost reduction are realized. Guide mirror 121 guides the user to place the eye to a correct image pickup position by reflecting an image of his/her own eye thereon.

Then, the image of the user's eye is picked up by image pickup element 124 through lens 123 and visible light eliminating filter 122. Preprocessing unit 125 acquires an image data component from the output signal from image pickup element 124, performs processing such as gain adjustment, which is required as the image data, and outputs as the eye image data of the user.

Pupil detection device 200 includes pupil candidate detection unit 210, pupil candidate retention unit 280, and pupil selection unit 290, and detects the position of the pupil and the radius thereof from the eye image, and outputs the same to authentication processing unit 140.

Authentication processing unit 140 cuts out an iris image from the eye image data based on the center coordinates and the radius of the pupil detected from pupil detection device 200. Then, authentication processing unit 140 converts the iris image into a specific iris code which indicates a pattern of the iris, and compares the same with the registered iris code to perform authentication operation.

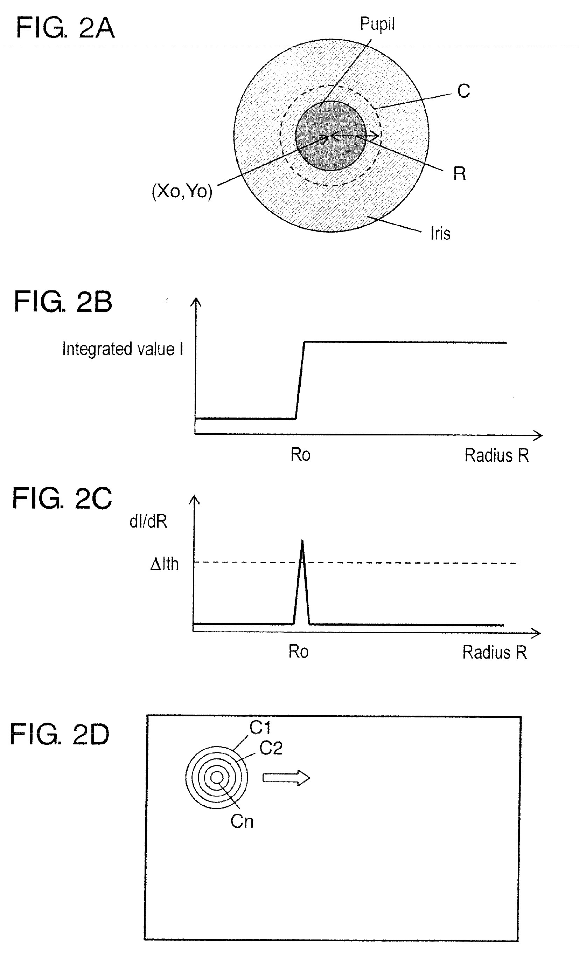

Subsequently, a method of detecting the pupil of pupil detection device 200 will be described. Fig. 2A to Fig. 2D are drawings for explaining a method of detecting the pupil performed by pupil detection device 200 in the first embodiment of the invention. Fig. 2A is a drawing showing an example of an image including a pupil. Fig. 2B is a drawing showing an integrated value with respect to the radius of the integrating circle. Fig. 2C is a drawing showing a value obtained by differentiating the integrated value by the radius of the integrating circle. Fig. 2D is a drawing showing integrating circles which move on the eye image.

The image including the pupil includes a low luminance area of a disk shape showing the pupil, and a middle luminance area of an annular shape indicating the iris outside thereof exiting therein as shown in Fig. 2A. Therefore, when the contour integral of the image data is performed along the circumference of integrating circle C having radius R about the positional coordinates (X₀, Y₀) at the center of the pupil, integrated value I changes stepwise on the border of pupil radius R₀, as shown in Fig. 2B. Therefore, by obtaining the radius of the integrating circle when value dI/dR obtaining by differentiating integrated value I by radius R exceeds a threshold (hereinafter, referred to as "difference threshold") ΔIth, pupil radius R₀ can be known.

Based on the idea described above, pupil detection device 200 detects the positional coordinates (X₀, Y₀) and pupil radius R₀. As shown in Fig. 2D, n integrating circles C₁-Cₙ having the same center coordinates and different radius are set on the eye image, and the image data located on the circumference is integrated with respect to each integrating circle Cᵢ (i=1-n). Realistically, an average value of the image data of the pixels located on the circumferences of each integrating circle Cᵢ is calculated. Alternatively, a certain number (m) of the pixels are selected from the pixels located on the circumference to add the image data thereof.

In the first embodiment, number n of the concentric integrating circles was assumed to be 20, and m=8 pixels were selected from the pixels located on the circumference of each integrating circle Cᵢ to add the image data to obtain integrated value I of the contour integral. When the center of integrating circles C₁-Cₙ is coincided with the center uf the pupil, as described above, integrated value Iᵢ with respect to each integrating circle Cᵢ changes stepwise. Therefore, when difference value ΔIᵢ with respect to radius R of integrated value Iᵢ is obtained, the values reach extremely large value at a point equal to pupil radius R₀.

However, since integrated value Iᵢ changes gently when the center of integrating circles C₁-Cₙ do not coincide with the center of the pupil, difference value ΔIᵢ is not a large value. Therefore, by obtaining integrating circle Cᵢ which has large difference value ΔIᵢ larger than difference threshold ΔIth, the position of the pupil and the radius thereof can be obtained.

Then, by moving integrating circles C₁-Cₙ to the respective positions on the eye image, the above-described operation is repeated. In this manner, by obtaining the center coordinates (X, Y) of integrating circle Cᵢ when difference value ΔIᵢ is large and radius R at that time, the positional coordinates (X₀, Y₀) of the pupil and pupil radius R₀ can be obtained.

However, depending on the image, there is a possibility that difference value ΔIᵢ shows a large value accidentally. In particular, the number n of integrating circles or the sum m of the number of the pixels to be selected on the respective integrating circles is reduced, the amount of calculation can be reduced, and hence pupil detection of high-speed is achieved. However, in contrast, the possibility that difference value ΔIᵢ shows a large value is accidentally increased, and hence the pupil detection accuracy is reduced. Therefore, luminance difference calculation unit 240 is provided on pupil detection device 200 for calculating difference Bᵢ between the maximum value and the minimum value of the luminance on the circumferences of each integrating circle Cᵢ, and, only when difference Bᵢ is smaller than predetermined threshold (hereinafter referred to as "luminance difference threshold) Bth, integrated value Iᵢ or difference value ΔIᵢ is considered to be effective, so that lowering of the pupil detection accuracy is prevented.

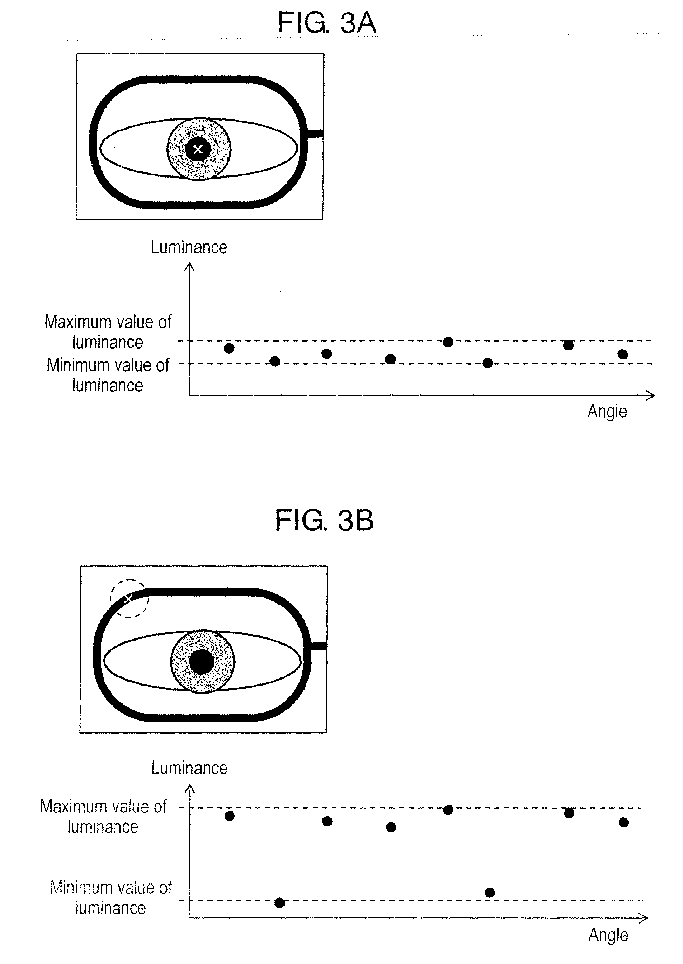

Fig. 3A and Fig. 3B are drawings for explaining the operation of luminance difference calculation unit 240. Fig. 3A is a drawing showing an example of an eye image when the integrating circle is positioned in the iris area and the luminance at the same moment, and Fig. 3B is a drawing showing an example of an eye image when the integrating circle is positioned on an eyeglass frame and luminance of the same moment.

When the centers of integrating circles C₁-Cₙ coincide with the center of the pupil, each integrating circle Cᵢ is positioned in an area with relatively uniform brightness such as inside the pupil area or inside the iris area, and hence variations in brightness of the image data on the circumference are small. Fig. 3A shows the integrating circle positioned in the iris area which is an annular middle luminance area. In this case, difference Bᵢ between the maximum value and the minimum value of the luminance on the circumference is small, and does not exceed luminance difference threshold Bth.

However, as shown in Fig. 3B for example, when the centers of integrating circles C₁-Cₙ are positioned on part of a black eyeglass frame, the luminance on the circumference is low on the eyeglass frame and high on the skin. Therefore, difference Bᵢ between the maximum value and the minimum value of luminance is large. In this manner, when difference Bᵢ between the maximum value and the minimum value of luminance on the circumference of each integrating circle Cᵢ is obtained, and only when difference Bᵢ is smaller than luminance difference threshold Bth, integrated value Iᵢ or difference value ΔIᵢ is determined to be effective, whereby erroneous determination such that the eyeglass frame is determined to be the pupil by mistake can be prevented, thereby preventing lowering of the pupil detection accuracy.

Luminance difference threshold Bth is preferably set to be slightly larger than estimated variations in luminance data on the circumference. Empirically, a value larger than the difference between the average luminance of the iris and the average luminance of the pupil, and smaller than the difference of the average luminance of the skin and the average luminance of the pupil is recommended. For example, in the case of the luminance having of 256 levels, an average luminance of the pupil is on the order of level equals 40, an average luminance of the iris is on the order of level equals 100, and an average luminance of the skin is on the order of level equals 200. Therefore, luminance difference threshold Bth may be set between 60 to 160.

As regards difference threshold ΔIth, integrated value I when the integrating circle is located on the pupil is about 40 x 8 = 320, and integrated value I when the integrating circle is located on the iris is about 100 x 8 = 800. Therefore, difference threshold ΔIth may be set to a value on the order of a half of the difference 480, that is, on the order of 240.

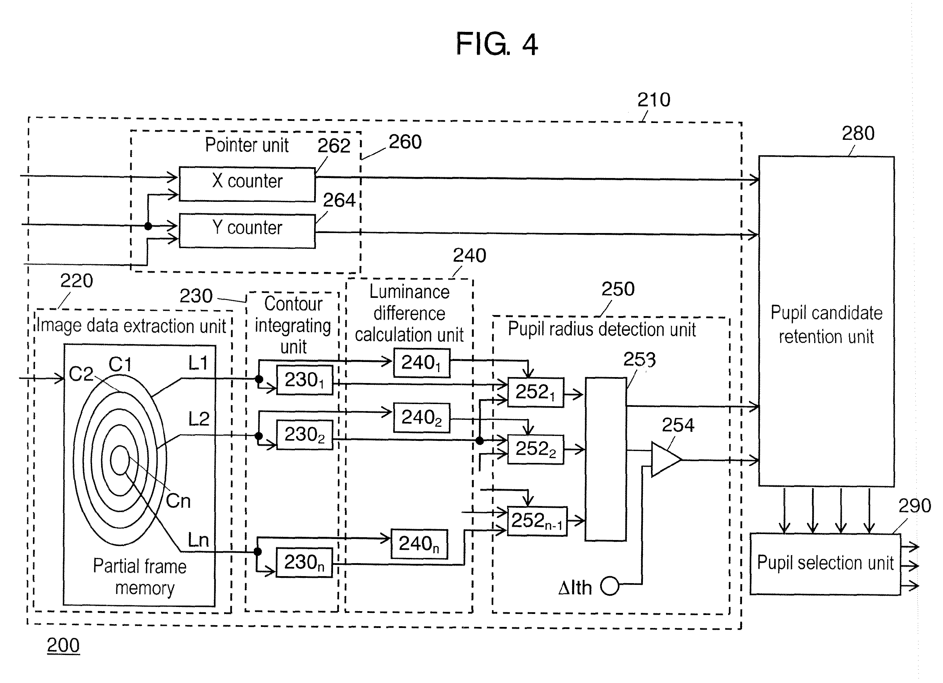

Fig. 4 is a circuit block diagram of pupil detection device 200 in the first embodiment of the invention. Pupil detection device 200 includes pupil candidate detection unit 210, pupil candidate retention unit 280, and pupil selection unit 290.

Pupil candidate detection unit 210 includes image data extraction unit 220 for setting integrating circles C₁-Cₙ on the eye image to extract the image data on the circumference of each integrating circle Cᵢ, contour integrating unit 230 performs contour integral on the extracted image data for each integrating circle Cᵢ, luminance difference calculation unit 240 for calculating difference Bᵢ between the maximum value and the minimum value of the image data for each integrating circle, and pupil radius detection unit 250 for obtaining difference value ΔIᵢ with respect to radius Rᵢ of integrated value Iᵢ and outputting difference value ΔIᵢ when maximum value ΔI of the difference value is larger than difference threshold ΔIth and radius R of the integrating circle, and pointer unit 260 showing center coordinates (X, Y) of integrating circles C₁-Cₙ.

Pupil candidate retention unit 280 is deemed to detect pupil candidate when pupil radius detection unit 250 outputs difference value ΔIᵢ larger than difference threshold ΔIth, and stores the positional coordinates (X, Y) of the plurality of pupil candidates and radius R, while pupil selection unit 290 selects one pupil from the plurality of pupil candidates.

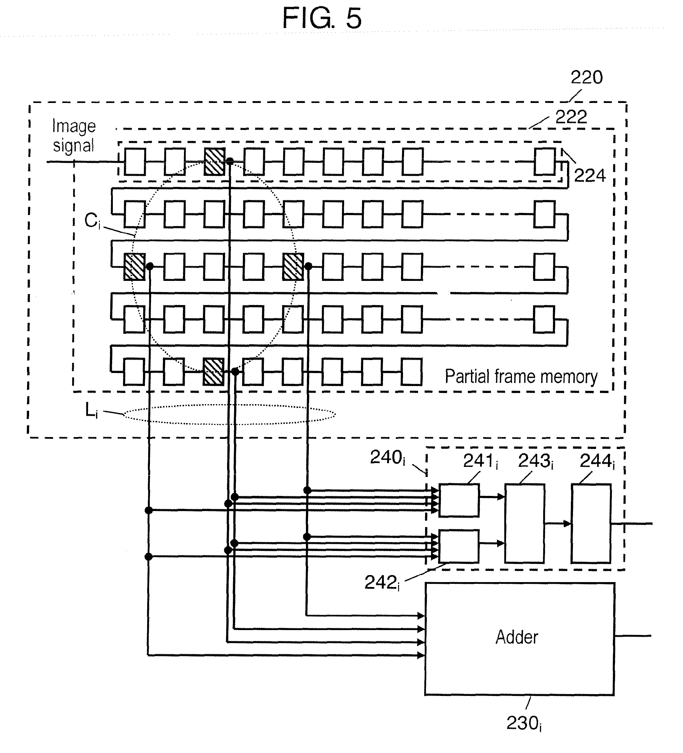

Fig. 5 is a circuit drawing of image data extraction unit 220. Fig. 5 also shows adder 230ᵢ corresponding to one of integrating circle Cᵢ and luminance difference calculator 240ᵢ. Image data extraction unit 220 includes partial frame memory 222, and drawing lines L for outputting the image data.

Partial frame memory 222 is a member including a plurality of line memories 224 of first-in first -out (FIFO type) connected in series. The image data is drawn from m pixels corresponding to integrating circle Cᵢ on the image by drawing line Lᵢ. For clarifying the illustration, Fig. 5 only shows one integrating circle Cᵢ, and four drawing lines Lᵢ for outputting the four image data located on the circumference thereof. However, in the first embodiment, eight data drawing lines are outputted from each of twenty integrating circles C₁-C₂₀.

Then, every time when image data is entered into partial frame memory 222 by one pixel, the entire image held in partial frame memory 222 is shifted by one pixel. Therefore, the image data outputted from drawing lines Lᵢ is also shifted by one pixel. In other words, when image data is entered into partial frame memory 222 by one pixel, integrating circles C₁-Cₙ move toward the right by the amount corresponding to one pixel on the eye image, and when the image data corresponding to one line is entered, integrating circles C₁-Cₙ move downward by the amount corresponding to one line on the eye image.

When image data which corresponds to one line is entered into partial frame memory 222, integrating circles C₁-Cₙ scan the entire eye image on the eye image. The center coordinates (X, Y) of the integrating circle at this time is shown by the outputs from X counter 262 and Y counter 264.

Contour integrating unit 230 is provided with independent adders 230₁-230ₙ for respective integrating circles C₁-Cₙ, m image data positioned on the circumference of each integrating circle Cᵢ are added, and each added result is outputted to the pupil radius detection unit 250 as integrated value Iᵢ.

Luminance difference calculation unit 240 is provided with luminance difference calculators 240₁-240ₙ provided independently for respective integrating circle C1-Cn, and each luminance difference calculator 240ᵢ includes maximum value detector 241ᵢ for detecting the maximum value of m pixel data positioned on the circumference of integrating circle Cᵢ, minimum value detector 242ᵢ for detecting the minimum value, subtracter 243ᵢ for calculating difference Bᵢ between the maximum value and the minimum value, and comparator 244ᵢ for comparing difference Bᵢ and luminance difference threshold Bth. Then, n compared results are outputted to pupil radius detection unit 250.

Pupil radius detection unit 250 is provided with subtracters 252₁-252ₙ₋₁, selector 253, and comparator 254. Subtracter 252ᵢ obtains the difference of integrated value Iᵢ of each integrating circle Cᵢ with respect to radius R. In other words, difference value ΔIᵢ between integrated values Iᵢ and Iᵢ₋₁ for integrating circles Cᵢ and Cᵢ₋₁ which are one-step different in radius out of integrating circles C₁-Cₙ is obtained. However, when difference Bᵢ between the maximum value and the minimum value of the image data with respect to integrating circle Cᵢ is larger than luminance difference threshold Bth, difference value ΔIᵢ is forcedly set to zero.

Then, selector 253 and comparator 254 output radius R of integrating circle C whose difference value ΔIᵢ is larger than difference threshold ΔIth to pupil candidate retention unit 280, and also output difference value ΔI to pupil candidate retention unit 280 as evaluated value J₀. At this time, when difference Bᵢ between the maximum value and the minimum value of the image data with respect to integrating circle Cᵢ is larger than luminance difference threshold Bth, subtracter 252ᵢ forcedly sets difference value ΔIᵢ to zero, and hence when difference Bᵢ is larger than luminance difference threshold Bth, radius Rᵢ is not outputted to pupil candidate retention unit 280.

As described based on Fig. 3, when the centers of integrating circles C₁-Cₙ coincide with the center of the pupil, difference Bᵢ between the maximum value and the minimum value of the pixel data does not exceed a certain limited value. However, when they do not coincide with the center of the pupil, difference Bᵢ is large. Therefore, by eliminating information when difference Bᵢ is larger than luminance difference threshold Bth, the possibility of erroneous detection can be reduced, thereby increasing the pupil detection accuracy.

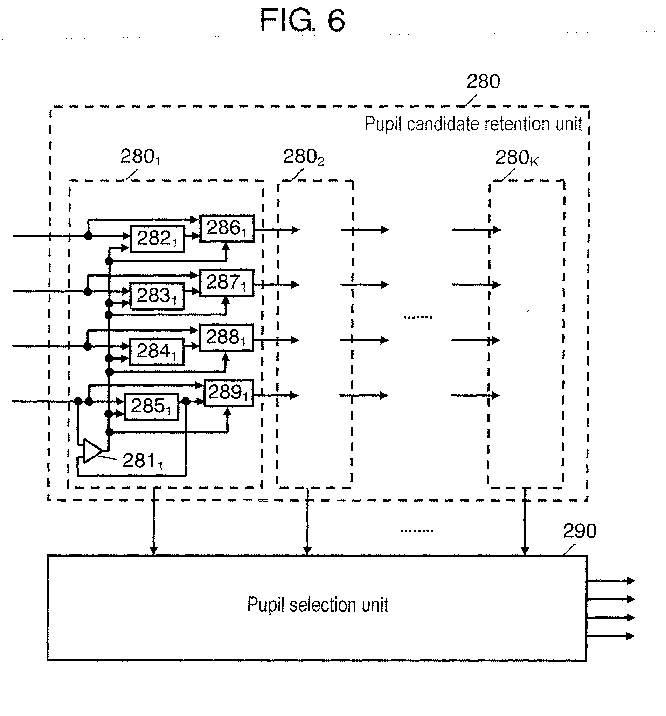

Fig. 6 is a circuit block diagram of pupil candidate retention unit 280 and pupil selection unit 290. Pupil candidate retention unit 280 includes a plurality of maximum value detectors 280₁-280ₖ connected in series. Each maximum value detector 280ᵢ includes registers 282ᵢ, 283ᵢ, 284ᵢ, and 285ᵢ for retaining maximum values of X-coordinate, Y-coordinate , radius R and evaluated value J, comparator 281ᵢ for comparing inputted evaluated value Jᵢ-₁ and evaluated value Jᵢ retained in register 285ᵢ, and selector 286ᵢ, 287ᵢ, 288ᵢ, and 289ᵢ for selecting any one of inputted X-coordinate, Y-coordinate, radius R and evaluated value J and retaining X-coordinate, Y-coordinate, radius R and evaluated value J.

Outputs X₀, Y₀ of X counter 262 and Y counter 264 indicating coordinates of the integrating circle as well as output Rₒ of pupil radius detection unit 250 are entered into first maximum value detector 280₁.

When evaluated value J₀ outputted from pupil radius detection unit 250 is larger than evaluated value J₁ retained by register 285₁, X-coordinate X₁, Y-coordinate Y₁, radius R₁, evaluated value J₁ retained in registers 282₁-285₁ to second maximum value detector 280₂ via selectors 286₁-289₁, and newly entered X-coordinate X₀ , Y-coordinate Y₀, radius R₀, evaluated value J₀ are retained in registers 282₁-285₁.

When evaluated value J₀ does not exceed evaluated value J₁, newly entered X-coordinate X₀, Y-coordinate Y₀, radius R₀, and evaluated value J₀ to second maximum value detector 280₂ via selectors 286₁-289₁.

When evaluated value J₁ outputted from first maximum value detector 280₁ is larger than evaluated value J₂ retained by register 285₂, second maximum value detector 280₂ outputs X-coordinate X₂, Y-coordinate Y₂, radius R₂, and evaluated value J₂ which have been retained by registers 282₂-285₂ thus far to third maximum value detector 280₃, and retains newly entered X-coordinate X₁, Y-coordinate Y₁, radius R₁ and evaluated value J₁ in registers 282₂-285₂. When evaluated value J₁ does not exceed evaluated value J₂, newly entered X-coordinate X₁, Y-coordinate Y₁, radius R₁, and evaluated value J₁ are outputted to third maximum value detector 280₃.

When evaluated value Jᵢ-₁ outputted from upstream maximum value detector 280ᵢ₋₁ is larger than evaluated value Jᵢ retained thus far, i^{th} maximum value detector 280ᵢ outputs data retained thus far to downstream maximum value detector 280ᵢ₊₁, and retains upstream data. When evaluated value Jᵢ₋₁ does not exceed evaluated value Jᵢ, the upstream data is outputted to the downstream side.

Consequently, X-coordinate X₁, Y-coordinate Y₁, radius R₁, evaluated value J₁ for the pupil candidate whose evaluated value is the largest are retained in first maximum value detector 280₁, and X-coordinate X₂, Y-coordinate Y₂, radius R₂, and evaluated value J₂ for the pupil candidate whose evaluated value is the second largest are retained in second maximum value detector 280₂, and X-coordinate Xᵢ, Y-coordi.nate Yᵢ, radius Rᵢ, and evaluated value Jᵢ for the pupil candidate whose evaluated value is the iₜₕ largest are retained in iₜₕ maximum value detector 280ᵢ.

Pupil selection unit 290 selects one pupil candidate, out of the plurality of pupil candidates retained in pupil candidate retention unit 280, which includes center positions of other pupil candidates in an area within a predetermined distance from the center position of its own, and outputs the positional coordinates and the radius to authentication processing unit 140 as the positional coordinates and the radius of the pupil. In this embodiment, the predetermined distance is 1.5 pixels. Therefore, pupil selection unit 290 counts the number of pupil candidates included in adjacent four pixels on the upper, lower, left and right sides of the positional coordinate (Xᵢ, Yᵢ) of the pupil candidate and four pixels at the obliquely adjacent positions, total eight pixels for each pupil candidate, and selects the pupil candidate which includes the largest number of pupil candidates as a real pupil.

If there are a plurality of pupil candidates which include the largest number of pupil candidates, the pupil candidate whose evaluated value Jᵢ is the largest is selected as the real pupil out of those pupil candidates. Consequently, the pupil selected by pupil selection unit 290 is the pupil having other pupil candidates therearound. Although pupil selection unit 290 may be configured by using a specific circuit which carries out the operation as described above, in this embodiment, a CPU (not shown) provided in authentication processing unit 140 is used for carrying out the above-described processing.

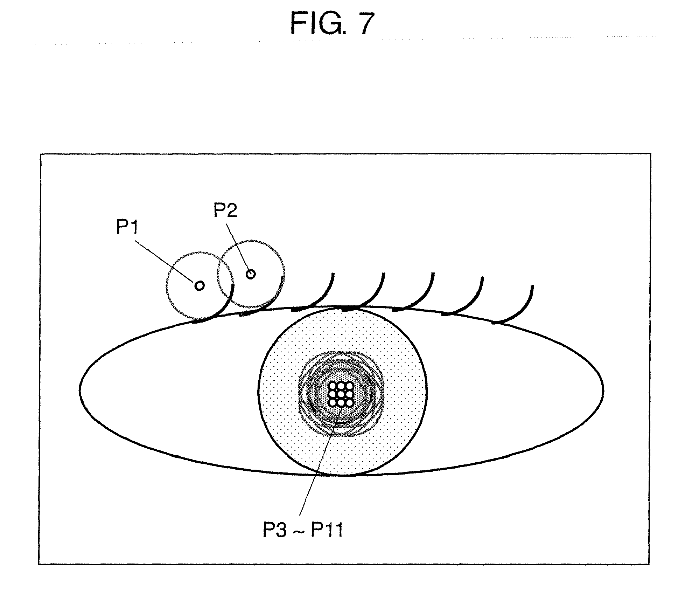

Fig. 7 is a drawing for explaining the operation of pupil selection unit 290. Pupil candidates P₁, P₂ are those where eyelash is detected erroneously as pupils, and pupil candidates P₃-P₁₁ are detected real pupils. In this manner, it is generally rare that the pupil candidates detected erroneously are in close formation, and there is a tendency that pupil candidates are in close formation around the real pupil. It depends on the detection accuracy of the pupil candidates, and the higher the detection accuracy is, the lesser the number of the pupil candidates in close formation becomes.

Since error about one pixel which depends on the image pickup element remains even though the accuracy is increased, there is a high possibility that the centers of other pupil candidates exist at the positions of adjacent pixels of the center position of the real pupil. Therefore, by selecting the pupil candidates having other pupil candidates therearound as the rear pupil, the erroneous detection such as to detect eyelash or the like as the pupil is eliminated, and hence the pupil detection accuracy can be improved.

Here, the number of the pixel positions for counting the number k of pupil candidates to be detected by pupil candidate retention unit 280, and the number of the pupil candidates existing around pupil selection unit 290 is preferably determined by detection accuracy of the pupil candidate, the estimated number of erroneously detected pupil candidate or the like. In this embodiment, the number of pixel positions for counting the number of the pupil candidates is set to the area including total eight pixels including four pixels on the upper, lower, left and right sides, and four pixels at the obliquely adjacent positions, considering the possibility that one each of-pupil candidate comes to the upper, lower, left and right sides of the real pupil position. Assuming that nine (one real pupil, one each on the upper, lower, left and right positions, and one each at the obliquely adjacent positions) pupil candidates are in close formation at the position of the real pupil, and there exists at most six pupil candidates which are erroneously detected, the number k of pupil candidates to be detected is set to 15.

In this manner, by detecting the plurality of pupil candidates from the eye image, and selecting the pupil candidate including the center positions of other pupil candidates at the pixel positions adjacent to the center position of the pupil candidate out of the plurality of pupil candidates, erroneous detection such as to detect the eyelash or the like as the pupil by mistake is eliminated and hence the pupil detection accuracy can be improved.

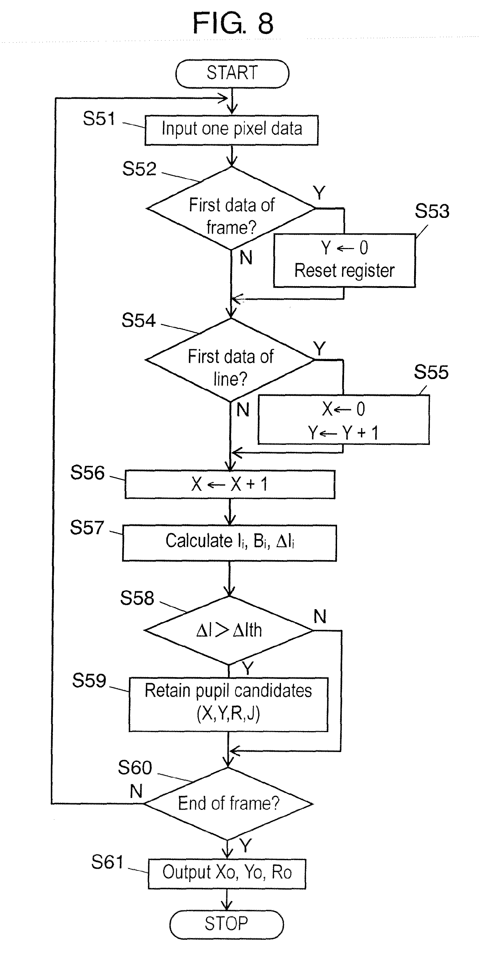

Subsequently, the operation of pupil detection device 200 will be described. In the following description, the eye image data is sequential scanning data, and one frame includes digital data of 480 lines x 640 pixels, for example. Fig. 8 is a flowchart showing the operation of pupil detection device 200 according to the first embodiment of the invention corresponds to one frame of the eye image.

Pupil detection device 200 acquires image data which corresponds to one pixel (S51). When the acquired image data is a first data of one frame (S52), Y councer 264 is reset and the respective registers 282-285 of the pupil candidate retention unit 280 are reset (S53). When acquired data is a first data of one line (S54), X counter 262 is reset and Y counter 264 is incremented (S55). Then, X counter 262 is incremented (S56).

Subsequently, acquired image data is acquired in partial frame memory 222. Then, m image data each time, and n x m image data are outputted from each integrating circle Cᵢ out of pixels corresponding n integrating circles C₁-Cₙ on the eye image. Then, adder 230ᵢ corresponding to each integrating circle Cᵢ calculates integrated value Iᵢ of each image data, and luminance difference calculator 240i calculates difference Bᵢ between the maximum value and minimum value of image data. Variation circle detection unit 250 calculates difference value ΔIᵢ of each integrated value Iᵢ. However, at this time, when difference Bᵢ is larger than luminance difference threshold Bth, difference value ΔIᵢ is forcedly set to zero (S57).

Then, comparator 254 compares difference value AIᵢ with difference threshold ΔIth (S58), and when difference value ΔIᵢ is larger than difference threshold ΔIth, pupil candidate retention unit 280 retains X counter 262, the Y counter 264, and radius Ro of integrating circle at this time as the pupil candidate and difference value ΔIᵢ as evaluated value Jo. At this time, pupil candidate retention unit 280 rearranges the pupil candidates in the descending order of the evaluated value, and k pupil candidates at maximum are retained (S59). Then, whether or not the acquired data is the data at the tail end of one frame is determined (S60), and if not, the procedure goes back to Step S51.

When the image data to be entered reaches the last pixel of one frame, pupil selection unit 290 calculates the number of other pupil candidates existing at the pixel positions of the center coordinates adjacent to the center coordinates thereof for the respective pupil candidates, and X-coordinate, Y-coordinate, and the value of the radius of the pupil candidate whose value is the largest are outputted to iris authentication processing unit 140 as X-coordinate Xo, Y-coordinate Yo, and pupil radius Ro of the real pupil (S61).

The series of operations from Step S51 to Step S60 are performed for each entry of the image data to partial frame memory 222 by the amount corresponding to one pixel. For example, when the frame frequency is 30Hz, and the eye image includes 640 x 480 pixels, the above-described series of operations are carried out within 1/(30 × 640 × 480) seconds. Then, when one pixel is inputted to partial frame memory 222, the integrating circle moves by an amount corresponding to one pixel on the image, and hence the integrating circle scans on the image once during the time when the image of one frame is entered. In this manner, the pupil is detected on the real time basis with respect to the image data picked up by image pickup unit 120 by using a circuit of relatively small scale.

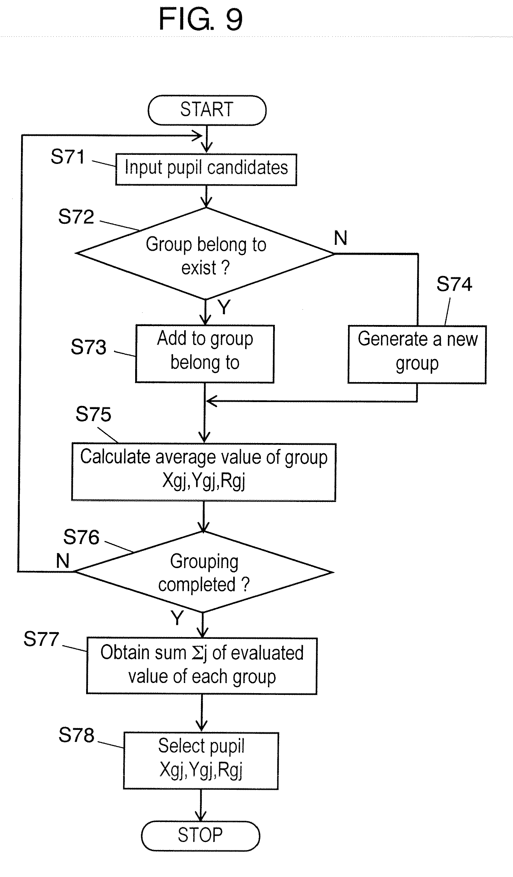

The method of selecting the pupil candidate, out of the plurality of pupil candidates, which includes center positions of other pupil candidates in the area within a predetermined distance from the center position of its own is not limited to the method described above. For example, a structure in which the plurality of pupil candidates are sorted into groups by grouping those close to each other as one group, and the real pupil is selected based on keys such as the group in which a large number of pupil candidates are included, or the group in which the sum of evaluated values of the pupil candidates are large may be employed. Fig. 9 is a flowchart for selecting the pupil out of the pupil candidates based on such an idea.

Pupil selection unit 290 acquires one pupil candidate first. X-coordinate, Y-coordinate, the radius, and the evaluated value of the acquired pupil candidate are represented respectively as Xi, Yi, Ri, and Ji (S71). Then, whether or not a group in which the differences between the values of pupil candidates Xi, Yi and Ri and the average values of groups Xgj, Ygj and Rgj (j is zero or a positive integer) is smaller than the predetermined thresholds Xth, Yth and Rth regarding each of X-coordinate, Y-coordinate and the radius exists is checked. In other words, whether or not the group which satisfies Xi-Xgj|<Xth, |Yi-Ygj| <Yth, | Ri-Rgj | <Rth exists is checked (S72).

If yes, the pupil candidate acquired in Step S71 is added to the group (S73). If not, a new group which only includes the pupil candidate acquired in Step S71 is generated (S74). Subsequently, recalculation of average values Xgj, Ygj and Rgj for the group to which the pupil candidate is added in Step S73 or the group newly generated in Step S74 (S75).

When the pupil candidates which are not grouped are remained, the procedure returns to Step S71 (S76). When the grouping is completed for every pupil candidate, sums ΣJ of evaluated values of the respective pupil candidates included in the group are obtained for the respective groups (S77) . Then, average values Xgj, Ygj and Rgj of X-coordinate, Y-coordinate, and the radius in the group whose sum Σj of the evaluated values is the largest is outputted to iris authentication processing unit 140 as the X-coordinate , Y-coordinate, and the radius (S78) .

According to the above-described method, although there remains instability such that the result of grouping may vary depending on the order of the pupil candidates in principle, since the pupil candidates which may be detected erroneously are isolated, and the pupil candidates which include the real candidate is in close formation, for example, if values of Xth, Yth are set to about 1/2 of the estimated radius of the pupil, there arises no problem in fact. According to this flow, the data processing is relatively easy and is suitable for the operation in high-speed.

Selector 253 of pupil radius detection unit 250 of this embodiment has a function to select the maximum value of difference value ΔIᵢ and radius R of integrating circle C at that time. However, pupil candidate retention unit 280 originally has a function to detect the maximum value. Therefore, it is also possible to employ selector 253 having a structure which outputs the output of subtracters 252₁-252ₙ₋₁ and the radius of the integrating circle simply by time division.

Although the number of the concentric integrating circles is twenty and the number of image data outputted from one integrating circle is eight in this embodiment, these numbers are preferably determined considering the detection accuracy, processing time, and the scale of the circuit in parallel.

According to the invention, the pupil detection device and the iris authentication apparatus which can detect the position of the pupil with high degree of accuracy and at high-speed is provided.

### Industrial Applicability

As the invention can provide the pupil detection device which can detect the position of the pupil with high degree of accuracy and at high-speed, it is effective for the iris authentication apparatus or the like which is used for personal authentication.

## Claims

1. A pupil detection device comprising:
a pupil candidate detection unit for detecting positions of pupil candidates which may be candidates of a pupil in an eye image;
a pupil candidate retention unit for retaining a plurality of positions of the pupil candidates detected by the pupil candidate detection unit; and
a pupil selection unit for selecting a pupil candidate, out of the pupil candidates retained in the pupil candidate retention unit, which includes center positions of other pupil candidates in an area within a predetermined distance from the center position of its own as a pupil.

2. A pupil detection device comprising:
an image data extraction unit for determining a plurality of concentric circles on an eye image as integrating circles respectively, and extracts image data of the eye image positioned on the circumferences of the integrating circles;
a contour integrating unit for integrating the image data extracted by the image data extraction unit along the respective circumferences of the integrating circles;
a pupil radius detection unit for detecting an integrating circle whose integrated value obtained from the contour integrating unit has changed stepwise with respect to the radius of the integrating circle out of the plurality of integrating circles;
a pupil candidate retention unit for retaining the center coordinates of the integrating circle detected by the pupil radius detection unit as a coordinate position of the pupil candidate, and
a pupil selection unit for selecting a pupil candidate, out of the pupil candidates retained in the pupil candidate retention unit, which includes center positions of other pupil candidates in an area within a predetermined distance from the center position of its own.

3. The pupil detection device of Claim 2, wherein the pupil radius detection unit outputs a difference of the integrated value of the contour integrating unit stepwise with respect to the radius of the integrating circle as an evaluated value, and the pupil candidate retention unit includes a plurality of maximum value detectors for detecting data having a maximum value out of input data connected in series and inputs the evaluated value in the pupil candidate retention unit, thereby retaining the pupil candidates in the descending order of the evaluated value.

4. The pupil detection device of Claim 3, wherein the maximum value detector comprises a register for retaining input data;
a comparator for comparing data retained in the register and the input data; and
a selector for selecting and outputting either the data retained in the register or the input data,
wherein the register retains the input data when the input data is larger than the retained data based on the output of the comparator, and
wherein the selector selects the data retained by the register when the input data is larger than the data retained by the register and selects the input data when the input data is smaller than the data retained by the register based on the output of the comparator.

5. The pupil detection device of Claim 2 wherein the pupil selection unit sorts the plurality of pupil candidates into groups by grouping those close to each other as one group, selects a group in which the largest number of pupil candidates are included or a group in which the sum of evaluated values of the pupil candidates are the largest out of the respective groups, and determines the pupil position in the selected group.

6. An iris authentication apparatus comprising the pupil detection device of any one of Claim 1 to Claim 5.
